# EUROPÄISCHE PATENTSCHRIFT

(11) **EP 2 364 105 B1**
(45) Veröffentlichungstag und Bekanntmachung des Hinweises auf die Patenterteilung: **14.03.2018**
(21) Anmeldenummer: 09752741.0
(22) Anmeldetag: 04.11.2009
(51) Int. Cl.: A61B 3/00

(54) **OPHTHALMOLOGISCHES MESSSYSTEM UND VERFAHREN ZU DESSEN KALIBRIERUNG UND/ODER JUSTIERUNG**
OPHTHALMOLOGICAL MEASURING SYSTEM AND METHOD FOR CALIBRATING AND/OR ADJUSTING THE SAME
SYSTÈME DE MESURE OPHTALMOLOGIQUE ET PROCÉDÉ PERMETTANT SON CALIBRAGE ET/OU SON AJUSTAGE

(30) Priorität: 04.11.2008 DE 102008055755; 30.09.2009 DE 102009043748
(43) Veröffentlichungstag der Anmeldung: 14.09.2011
(73) Patentinhaber: Carl Zeiss Meditec AG, 07745 Jena (DE)
(72) Erfinder: ANTKOWIAK, Gerard, 07743 Jena (DE); HACKER, Martin, 07745 Jena (DE); KOSCHMIEDER, Ingo, 07743 Jena (DE); BERGNER, Roland, 07745 Jena (DE); EBERSBACH, Ralf, 04626 Schmölln (DE); PABST, Thomas, 07646 Stadtroda (DE); HOFMANN, Eberhard, 07646 Bollberg (DE); DUBNACK, Steffen, 07745 Jena (DE); GÜNTZSCHEL, Michael, 07751 Jena (DE)
(74) Vertreter: Beck, Bernard
(86) Internationale Anmeldenummer: PCT/EP2009/007891
(87) Internationale Veröffentlichungsnummer: WO 2010/051974

(56) Entgegenhaltungen:
- WO-A1-02/11612
- DE-U1-202005 021 287
- US-A1- 2008 204 656

## Beschreibung

Die vorliegende Erfindung betrifft ein ophthalmologisches Messsystem zur Gewinnung der biometrischen Daten eines Auges, welches über die zur Überprüfung der Funktionalität und des Kalibrierungszustandes erforderlichen Kalibrier- und Prüfmittel verfügt. Die Kalibrierung und/oder Justierung derartiger optischer Geräte erfolgt hierbei unter Zuhilfenahme mindestens eines derartigen Kalibrier- und Prüfmittel.

Die bei ophthalmologischen Meßsystemen verwendeten Kalibrier- und Prüfmittel werden hierbei auch als Referenzobjekte und insbesondere als sogenannte Prüfaugen bezeichnet.

Bei bestimmten technischen und insbesondere medizintechnischen Geräten ist eine wiederkehrende Kalibrierung und Justierung im Routineablauf notwendig, um die erforderliche Qualität, Funktionstüchtigkeit und Sicherheit der Geräte gewährleisten zu können. Die zunehmend angestrebte Erhöhung der Messgenauigkeit der technischen Geräte steht dabei im Widerspruch zur Möglichkeit der einfachen und reproduzierbaren Genauigkeit der Kalibrier- und Prüfmittel.

Meist sind Benutzer ophthalmologischer Geräte angehalten Kalibrier- und Prüfmittel in vorgegebenen Intervallen bzw. vor Messungen am Patienten auf das Gerät aufzustecken, zu vermessen und die erhaltenen Messwerte mit den Vorgaben zu vergleichen, um dessen Funktionalität und Kalibrierungszustand zu prüfen. Dies ist insbesondere bei Geräten zur Biometrie, Keratometrie, Topographie des Auges oder auch bei Scheimpflug-Kameras der Fall. Hierbei müssen komplexe Lichtstrukturen, wie Scheibchen, Ringe, Schlitze o. ä. unter großen bzw. sehr verschiedenen Winkeln, mitunter auch gleichzeitig auf das Auge projiziert werden.

Nach dem Stand der Technik sind sowohl ophthalmologische Messsysteme zur Bestimmung der biometrischen Daten eines Auges als auch Kalibrier- und Prüfmittel zur Überprüfung der Funktionalität und des Kalibrierungszustandes in Form von Testaugen hinreichend bekannt, wobei hierzu Kalibrier- und Prüfmittel in Form von Maßstabs- oder Oberflächenverkörperungen verwendet werden.

Deshalb werden in der Ophthalmologie die Kalibrier- und Prüfmittel in Form von Prüfaugen vorab mit Prüfnormalen ausgemessen, bewertet und entsprechend gekennzeichnet. Nach dem bekannten Stand der Technik erhalten die Testaugen lediglich eine für den Nutzer ohne technische Hilfsmittel lesbare Angabe, in Form von numerischen Werten oder physikalischen Größenangaben. Dabei sind bei manchen, optischen Gerät die Daten der zu verwendenden Testaugen hinterlegt. Für die Verwendung anderer als der gespeicherten Testaugen ist eine entsprechende Anpassung der Software des optischen Gerätes erforderlich.

Bei anderen optischen Geräten sind diese Daten nicht hinterlegt. Der Betreiber des zu kalibrierenden und/oder zu justierenden, optischen Gerätes hat dann während der Kalibrierung oder Justierung die Aufgabe, die auf dem Testaugen aufgedruckten Ziffernwerte mit der Messwerten des optischen Gerät bei Vermessung des Testauges manuell zu vergleichen. Erfahrungsgemäß kann es in der täglichen Praxis durch mangelnde Sorgfalt hierbei zu Fehlentscheidungen kommen. Diese Fehlentscheidungen haben zwangsläufig falsche Messergebnisse zur Folge und können letztlich zu Fehldiagnosen und sogar Fehlbehandlungen führen. Fehlentscheidungen oder Verwechslungen aufgrund mangelnder Sorgfalt können unter anderem auch in einem angrenzenden Fachgebiet zu erheblichen Problemen führen.

So beschreibt die DE 195 04 465 A1 ein Kalibrier- und Prüfmittel für optische Augenlängenmessgeräte, insbesondere zur interferometrische Messungen. Als Kalibrier- und Prüfmittel werden hierzu transparente Kugeln eines Durchmessers von etwa 16 mm und einer Brechzahl von etwa 2 verwendet. Dadurch entspricht der Krümmungsradius der Oberfläche des Prüfkörpers in etwa dem der Augenhornhaut, so dass der Strahlengang im Meßsystem wie bei der Messung im natürlichen Auge verläuft. In einer bevorzugten Ausgestaltung weist der Prüfkörper eine definiert verringerte Transmission auf, um die Transmission und/oder Reflexivität den Verhältnissen am Auge besser anzupassen. Durch Veränderung der Reflexivität der Beschichtungen der Vorderseite und/oder der Rückseite des Prüfkörpers oder deren Transparenz können unterschiedliche menschliche Augen nachempfunden werden.

Ein weiteres Kalibrier- und Prüfmittel für optische Augenlängenmessgeräte wird in der DE 199 36 571 B4 beschrieben. Dieses besteht aus zwei im Beleuchtungsstrahlengang angeordneten, entgegen gesetzt orientierten Plankonvexlinsen, zwischen denen sich ein Neutralfilter mit einer definierten Transmission befindet. Hiermit wird ein Prüfkörper zur Verfügung gestellt, der trotz seines relativ einfachen Aufbaus universell für optische Augenlängenmessgeräte anwendbar ist. Zur Reduzierung unerwünschter Reflexe an der Planplatte wird die gesamte Anordnung unter einem Winkel zwischen 10 und 20 Grad benutzt. Durch den Einsatz eines anderen Graufilters oder der Variation seiner Dicke lassen sich weitere Prüfkugel dimensionieren, die unterschiedliche Trübungswerte durch Augenkatarakt simulieren. Vorteilhaft kann durch Variation der Verkippung im Strahlengang ein genaues Absorptionsmaß einstellen, da eine Verkippung um etwa Grad eine Transmissionskorrektur von etwa 20-25% bedeutet.

Nach dem Stand der Technik sind zu ophthalmologischen Messsystemen zur Bestimmung der biometrischen Daten eines Auges nur Lösungen bekannt, bei denen die erforderlichen Kalibrier- und Prüfmittel als separate optische Elemente ausgeführt sind und in regelmäßigen Zeitabständen zur Überprüfung der Funktionalität und des Kalibrierungszustandes verwendet werden.

Zur Bestimmung der biometrischen Daten eines Auges gibt es eine Reihe bekannter Verfahren und Messgeräte. Beispielsweise ist es erforderlich vor einem operativen Eingriff zum Austausch der Augenlinse bei Vorliegen einer Linsentrübung (Katarakt) verschiedene biometrische Parameter des Auges zu bestimmen. Um ein möglichst optimales Sehvermögen nach der Operation zu gewährleisten, ist es notwendig diese Parameter mit entsprechend hoher Genauigkeit zu bestimmen. Die Auswahl einer geeigneten Ersatzlinse anhand der ermittelten Messwerte erfolgt anhand etablierter Formeln und Berechnungsmethoden.

Die wichtigsten, zu ermittelnden Parameter sind u. a. die Achslänge (Abstand bis zur Retina), die Hornhautkrümmung und -brechkraft, sowie die Länge der Vorderkammer (Abstand bis zur Augenlinse). Diese Messwerte können nacheinander an verschiedenen ophthalmologischen Geräten oder mit Hilfe speziell optimierter, biometrischer Messgeräte gewonnen werden.

Zur Ermittlung dieser Parameter haben sich neben Ultraschallmessgeräten vor allem optische Messgeräte auf der Basis von Kurzkohärenzinterferometrieverfahren durchgesetzt. Bei diesen auf Kurzkohärenzinterferometrie basierenden Verfahren werden Tiefenprofile oder zweidimensionale Tiefenschnittbilder von Streupotentialen, insbesondere von Streuungen an Strukturübergängen, dargestellt. Als kurzkohärente Messverfahren haben sich hierbei das sogenannte OCDR-Verfahren (OCDR = optical coherence domain reflectometry) und das sogenannte OCT-Verfahren (OCT = optical coherence tomography) durchgesetzt.

Beim OCDR wird zeitlich inkohärentes Licht mit Hilfe eines Interferometers zur Erfassung von Tiefenprofilen an reflektiven und streuenden Strukturen und darauf basierenden Abstandsmessungen verwendet. Beim OCT wird darüber hinaus, wie in der US 5,321,501 beschrieben, mittels einer Strahlablenkung eine Bildgebung an den reflektiven bzw. streuenden Strukturen realisiert. Solche Systeme, wie auch Scheimplugkamerasysteme, sind beispielsweise geeignet, um biometrischer Daten aus Bildinformationen zu gewinnen, wie beispielsweise der Dimensionen der Augenvorderkammer, wie sie beispielsweise für die Anpassung phakischer Intraokularlinsen benötigt werden.

So beschreibt die US 7,322,699 B2 ein Kombinationsgerät zur berührungslosen Bestimmung von biometrischen Daten, wie Achslänge, Vorderkammertiefe sowie Hornhautkrümmung. Mit der beschriebenen Lösung ist es möglich, ausgehend von der Messung der erforderlichen Daten, über die Berechnung bis hin zur Auswahl der zu implantierenden Intraokularlinse IOL, mit nur einem Gerät durchzuführen. Erhöhte Belastungen des Patienten durch mehrmaliges Platzieren und Messen an verschiedenen Geräten können dadurch ebenso vermieden werden wie Datenverluste oder Datenverfälschungen durch Übertragung der Messwerte zwischen verschiedenen Geräten.

Ein Kombinationsgerät zur berührungslosen Bestimmung von Achslänge, Vorderkammertiefe sowie Hornhautkrümmung des Auges, sowie der Berechnung und Auswahl einer zu implantierenden Intraokularlinse IOL wird in der DE 198 57 001 A1 beschrieben. Insbesondere vor einer Katarakt-Operation, aber auch bei der Verlaufskontrolle der Schulmyopie und der Aniseikoniebestimmung müssen diese Messgrößen bestimmt werden, die auch für die Auswahl der zu implantierenden Intraokularlinse IOL wichtig sind. Die ermittelten Messgrößen werden in Formeln eingesetzt, welche die optische Wirkung der IOL errechnen. Je nach eingesetztem Gerätetyp kann es zu unterschiedlichen Fehlern kommen, welche die Auswahl der IOL beeinflussen.

In der klinischen Praxis war es bisher üblich, diese Größen zumindest mittels zweier Geräte (z. B. Ultraschall a-scan und automatisches Keratometer) zu messen. Da die Berechnung der IOL nunmehr mittels einer Geräteanordnung durchgeführt werden kann, entfallen auch Datenverluste oder Datenverfälschungen bei der Übertragung der Messwerte von verschiedenen Geräten zu dem Rechner, der die Berechnung der IOL durchführt. Der auf einem kurzkohärenten Verfahren basierende IOLMaster® der Carl Zeiss Meditec AG stellt ein optisches Messgerät, nach dem beschriebenen Lösungsprinzip dar.

Üblicherweise ist zur Kalibrierung und/oder zur Funktionsüberprüfung der Messfunktionen derartiger Geräte eine mitgelieferte Prüfkugel in regelmäßigen Abständen zu vermessen.

Dazu wird beispielsweise ein Halter mit einer Prüfkugel in neben der Kinnauflage befindliche Löcher gesteckt. Auf den Prüfkugeln sind die entsprechenden Sollwerte und die Toleranzen vermerkt, die der Überprüfung des Kalibrierzustandes dienen. Das Gerät sollte nur in Betrieb genommen werden, wenn die Messungen Ergebnisse liefern, die den auf der Prüfkugel angegebenen Sollwerten innerhalb der ebenfalls angegebenen Toleranzen entsprechen. Nach erfolgter Messung sind die Prüfkugeln wieder zu entfernen und sicher zu verstauen, um Beschädigungen und/oder Verschmutzungen zu verhindern.

In der US 2007/0291277A1 wird ein ophthalmologisches System beschrieben, welches aus einem optischen Kohärenztomographie-System (OCT), einem Fundus-Erfassungssystem, einem Iris-Erfassungssystem, einer motorisierten Kinnauflage, internen Teststrukturen und einer Fixiermarken-Einheit besteht. Dabei sind die internen Teststrukturen, die im Wesentlichen als beabstandete Flächen sowie als Fadenkreuz oder als horizontale bzw. vertikale Balkenstrukturen ausgebildet sind, zur Überprüfung einzelner Funktionalitäten vorgesehen. Allerdings sind mit diesen internen Teststrukturen nur interne Funktionsprüfungen und Kalibrierungen des OCT-Systems (optical coherent tomography) bzw. des zur Fundus-Erfassung verwendeten LSLO-Systems (line scanning laser ophthalmoscope) möglich, also insbesondere nicht solche unter Berücksichtigung aller Optikkomponenten auf dem ganzen Strahlweg zum und vom Patientenauge. Deshalb ist in der Schrift US 2007/0291277 A1 neben der internen Teststruktur auch weiterhin ein konventionelles Testauge beschrieben, welches lediglich für Kalibrierungen vorgesehen ist und dazu manuell aufgesetzt werden muss.

Die Schrift WO 2006/128596 A1 beschreibt in diesem Zusammenhang ein Mikrokeratom zum Einsatz in der Ophthalmologie, insbesondere beim LASIK-Verfahren. Bei dem sogenannten LASIK-Verfahren wird die Hornhaut des Auges quer zur optischen Achse eingeschnitten, sodass ein Hornhautdeckelchen, auch Flap genannt, entsteht. Nach Aufklappen des Flap erfolgt die Ablation der darunter liegenden Stroma der Hornhaut. Somit kann die Hornhaut je nach Fehlsichtigkeit entsprechend modelliert werden. Nach erfolgter Korrektur wird der Flap wieder in seine ursprüngliche Lage zurückgeklappt. Der Flap saugt sich dabei von selbst wieder fest und verwächst, ohne angenäht werden zu müssen. Für das Einschneiden der Hornhaut des Auges werden Mikrokeratome verwendet, die in der Regel Austauschkomponenten aufweisen, die vom Benutzer in Abhängigkeit von der anstehenden Operation ausgewählt werden. Solche Austauschkomponenten sind insbesondere der Schneidkopf und der Saugring. Unterschiedliche Austauschkomponenten haben unterschiedliche Auswirkungen auf die Durchführung des chirurgischen Eingriffs. Bei der hier beschriebenen Lösung sind die Austauschkomponenten jeweils mit einer Kennung versehen, die die Identifikationsmerkmale der jeweiligen Austauschkomponente beschreibt. Mit einem Lesegerät können die Kennungen ausgelesen und verwertet werden. Die Kennungsdaten können dem Benutzer zum Beispiel auf einem Bildschirm direkt angezeigt werden und/oder einer Berechnung zur Steuerung des Mikrokeratomsystems zugrunde gelegt werden. Bei dem hier beschriebenen System sind für die Kennung der Austauschkomponenten bevorzugt Barcodes vorgesehen. Die hier beschriebene Lösung setzt die Verwendung eines zusätzlichen Lesegerätes in Form eines entsprechenden Barcodescanners voraus und ist weder zur Kalibrierung und/oder Justierung eines Gerätes vorgesehen, noch ist es dafür geeignet. Dokumente WO 02/11612 und US 2008/204656 offenbaren ophthalmologische Messsysteme gemäß des Oberbegriffs des Anspruchs 1. Nachteilig bei diesen Lösungen wirkt sich aus, dass die Verwendung externer Testaugen für den Benutzer umständlich ist und eine wesentliche Fehlerquelle in sich birgt. Verständlicherweise ist das Aufbewahren, Aufstecken, Vermessen des Testauges, sowie das Ablesen und Vergleichen der Kalibrierdaten nicht benutzerfreundlich.

Der vorliegenden Erfindung liegt die Aufgabe zugrunde ein ophthalmologisches Messsystem, insbesondere zur Bestimmung der biometrischen Daten zu entwickeln, welches die im Stand der Technik erwähnten Nachteile nicht aufweist. Dabei soll das Kalibrier- und Prüfmittel mindestens eine Teststruktur aufweisen und für die Anwendung in ophthalmologischen Geräten mit Strukturprojektion, insbesondere Keratometern geeignet sein. Dabei soll die Kalibrierung und/oder Justierung für das Bedienpersonal wesentlich vereinfacht und dabei die nach dem Stand der Technik bekannten Fehlerquellen beseitigt werden. Erfindungsgemäß wird die Aufgabe durch das erfindungsgemäße ophthalmologische Messsystem gemäß des Anspruchs 1 gelöst. Dazu wird das zu verwendende Kalibrier- und Prüfmittel in die Messposition gebracht, dessen individuellen physikalischen Daten von dem zu kalibrierenden und/oder zu justierenden, ophthalmologischen Messsystem ausgelesen, dessen physikalischen Eigenschaften von dem zu kalibrierenden und/oder zu justierenden, ophthalmologischen Messsystem gemessen, die ausgelesenen physikalischen Daten des Kalibrier- und Prüfmittels mit dessen gemessenen physikalischen Eigenschaften verglichen werden und daraus Erkenntnisse zum Kalibrier- und/oder Justierzustand sowie eine Entscheidung zur weiteren Verwendung des ophthalmologischen Messsystems abgeleitet.

Bevorzugte Weiterbildungen und Ausgestaltungen sind Gegenstand der abhängigen Ansprüche.

Obwohl die vorliegende Erfindung ein ophthalmologisches Messsystem, insbesondere zur Bestimmung der biometrischen Daten betrifft, ist die erfinderische Lösung der Integration von Kalibrier- und Prüfmitteln auch für andere ophthalmologische, dermatologische oder auch andere Geräte, bei denen in regelmäßigen Abständen Kalibrierungen und/oder Funktionsüberprüfungen erforderlich sind, geeignet. Dies schließt auch Messsysteme als Teile ophthalmologischer Therapiesysteme ein, beispielsweise solcher zur Photokoagulation an der Netzhaut oder Laserbearbeitung zur Refraktionskorrektur.

Die Erfindung wird nachfolgend anhand von Ausführungsbeispieles näher beschrieben. Dazu zeigen.
- Figur 1:: einen IOLMaster® mit, in die Kopfstütze integriertem Prüfauge,
- Figur 2:: eine Vorrichtung zur Aufnahme mindestens eines Prüfauges mit einer Abdeckklappe und Mittel zum Reinigen des Prüfauges,
- Figur 3:: ein ophthalmologisches Messsystem mit, in den Gerätemesskopf integriertem Prüfauge,
- Figur 4:: das erfindungsgemäße Kalibrier- und Prüfmittel in Form zweier, in einem Halter angeordneten Prüfaugen,
- Figur 5:: die Prüfaugen nach Figur 4 in Verbindung mit einem auf interferometrischen Messverfahren basieren, ophthalmologischen Gerät und
- Figur 6:: die Prüfaugen nach Figur 4 in Verbindung mit einem auf der Wellenfrontanalyse basieren, ophthalmologischen Gerät.

Das erfindungsgemäße ophthalmologische Messsystem, insbesondere zur Bestimmung der biometrischen Daten eines Auges besteht mindestens aus Mitteln zur Beleuchtung eines Auges mit Licht und Mitteln zur Erfassung und Auswertung zurück gestreuter oder reflektierter Lichtanteile, sowie einer Steuereinheit. Die für eine Kalibrierung sowie für die Überprüfung der Funktion und des Kalibrierungszustandes erforderlichen Kalibrier- und Prüfmittel sind hierbei in das ophthalmologische Messsystem integriert, wofür das ophthalmologische Messsystem über mindestens eine Vorrichtung zur Aufnahme und Ausrichtung der Position mindestens eines das Kalibrier- und Prüfmittels in Bezug auf die Mittel zur Erfassung zurück gestreuter oder reflektierter Lichtanteile verfügt.

Als Mittel zur Erfassung zurück gestreuter oder reflektierter Lichtanteile werden Photodetektoren oder auch Kameras verwendet.

Das ophthalmologische Messsystem kann hierbei insbesondere über Mittel zur gleichzeitigen Beleuchtung eines Auges mit Licht aus einer oder mehreren Lichtquellen und unter unterschiedlichen, insbesondere großen Winkeln zur Sehachse des Auges von >5°, >10° oder sogar >15° verfügen, wie es beispielsweise bei ophthalmologischen Geräten zur Keratometrie oder Topographie erforderlich ist.

Vorzugsweise verfügt das Kalibrier- und Prüfmittel über mindestens eine gekrümmte Oberfläche als Teststruktur. Mögliche Teststrukturen sind hierbei:
- Testflächen, insbesondere -sphären, für die Bestimmung des Krümmungsradius an Kornea und/oder Augenlinse,
- Abstandsstrukturen zur Kalibrierung von Teilstreckenmessungen, wie Achslänge oder Vorderkammertiefe,
- Reflektoren mit bestimmten Reflexionsgraden (Abschwächer) zur Sensitivitätsbestimmung und
- Testmuster für Auflösungstests und laterale Kalibrierung von Fundus- oder Vorderkammerabbildungen.

Die Vorrichtung zur Aufnahme mindestens eines Kalibrier- und Prüfmittels verfügt über eine Vorrichtung, mit denen das Kalibrier- und Prüfmittel mechanisch bzw. halb- oder vollautomatisch in die für die Messung erforderliche Position bewegt wird. Eine Möglichkeit der halbautomatischen Positionierung ist beispielsweise eine manuelle Rückstellung des das Kalibrier- und Prüfmittels in die Aufnahmevorrichtung, nach einem elektromechanisch ausgelösten Ausklappen oder Ausfahren des Kalibrier- und Prüfmittels aus der Aufnahme mittels einer Feder. Hiermit lässt sich beispielsweise eine Positionierung des das Kalibrier- und Prüfmittels mit einem Minimum an elektromechanischen Komponenten realisieren.

Vorzugsweise erfolgt die Bewegung des Kalibrier- und Prüfmittels aber motorisiert und vollautomatisch in die für die Messung erforderliche Position, d. h. an eine vorgegebene oder signaloptimierte Position nahe der Nominalposition des Patientenauges. Dazu verfügt das ophthalmologische Messsystem über eine entsprechende Antriebseinheit.

Es ist aber auch möglich, dass das ophthalmologische Messsystem über Mittel verfügt, mit denen es auf das Kalibrier- und Prüfmittel mechanisch bzw. halb- oder vollautomatisch ausgerichtet wird, oder dass das ophthalmologische Messsystem über Mittel verfügt, mit denen die Messstrahlen des ophthalmologischen Messsystems auf das fest angeordnete Kalibrier- und Prüfmittel umgelenkt bzw. ausgerichtet werden.

Nach der Ausrichtung ist gegebenenfalls eine relative Feinpositionierung zwischen dem Kalibrier- und Prüfmittel und dem Gerätemesskopf erforderlich, damit die Messstrahlen ausgehend von einer Beleuchtungseinheit nach Reflexion oder Rückstreuung am das Kalibrier- und Prüfmittel optimal bzw. in genügendem Maße in das Messmodul einfallen.

Dazu können auch Sensoren, wie Photoempfänger mit Lochblenden, im Kalibrier- und Prüfmittel oder in der Aufnahme hinter dem Kalibrier- und Prüfmittel vorgesehen sein, die ein geeignetes Rückkopplungssignal für eine automatische Feinpositionierung liefern können oder aber auch Signale zur Feststellung von Laserleistungen oder auch Transmissionsveränderungen am das Kalibrier- und Prüfmittel, beispielsweise infolge von Verschmutzungen.

Weitere vorteilhafte Ausgestaltungen sind darin zu sehen, dass die Vorrichtung zur Aufnahme mindestens eines Kalibrier- und Prüfmittels über eine Schnittstelle zur Identifizierung unterschiedlicher Kalibrier- und Prüfmittel verfügt.

Dabei ist die Vorrichtung zur Aufnahme mindestens eines Kalibrier- und Prüfmittels so ausgebildet, dass verschiedene Kalibrier- und Prüfmittel aufgenommen werden können, um diese für die Messaufgaben teil- oder vollautomatisch zu positionieren. Die verwendeten Kalibrier- und Prüfmittel müssen hierbei nicht von ein und demselben Typ sein, sondern können beispielsweise auch für verschiedene Typen von ophthalmologischen Geräten und deren unterschiedliche Messanforderungen vorgesehen sein. Dazu ist die Vorrichtung zur Aufnahme mindestens eines Kalibrier- und Prüfmittels immer gleich gestaltet und ermöglicht so einen universellen Einsatz. Über die an der Vorrichtung vorhandene universelle Schnittstelle können die verschiedenen Kalibrier- und Prüfmittel identifiziert werden. Dies hat außerdem den Vorteil einer kostengünstigen Herstellung durch baugleiche Serien, niedriger Entwicklungskosten und einfacherer Montage und Reparatur.

In einer besonders vorteilhaften Ausgestaltung ist zur mechanischen bzw. halb- oder vollautomatischen Ausrichtung des Kalibrier- und Prüfmittels auf das ophthalmologische Messsystem und zur Positionierung einer Kopfstütze und/oder Kinnauflage nur eine Antriebseinheit vorhanden. Von dieser Antriebseinheit wird für die Positionierung der Kopfstütze und/oder Kinnauflage eine lineare und für die Ausrichtung des Kalibrier- und Prüfmittels eine lineare und/oder rotierende Bewegung realisiert. Dazu ist die Vorrichtung zur Aufnahme mindestens eines Kalibrier- und Prüfmittels ist hierbei an der Kopfstütze oder am Gerätemesskopf selbst angeordnet. Weiterhin ist es vorteilhaft, dass das Mittel zur Aufnahme mindestens eines Kalibrier- und Prüfmittels so ausgebildet ist, dass das Kalibrier- und Prüfmittel austauschbar ist.

Die meisten ophthalmologischen Messsysteme verwenden für die Patientenfixierung eine Kopfstütze mit Kinnauflage, wobei diese oft bereits mit einer motorischen Höhenverstellung versehen sind. Die Automatik in dieser Ausführungsform beinhaltet die Verwendung des bereits vorhandenen Motors für die Kinnauflage. Durch eine Erweiterung des Verfahrweges wird zusätzliche Bewegung für ein Prüfauge möglich. Dieser Mechanismus setzt die Linearbewegung der Kinnstütze in eine Rotation nur für das Testauge um. Somit besteht die Einheit aus einer motorisierten Kinnstütze, einem verlängerten Verstellweg, einem Mechanismus um den nötigen Verstellweg zu minimieren, einer Möglichkeit ein Prüfauge oder Messnormal an dem Mechanismus zu befestigen, einer Steuereinheit, einiger Elemente um die korrekte Position sicherzustellen und optional mit einer Sicherheitseinrichtung um keinen Patienten zu gefährden.

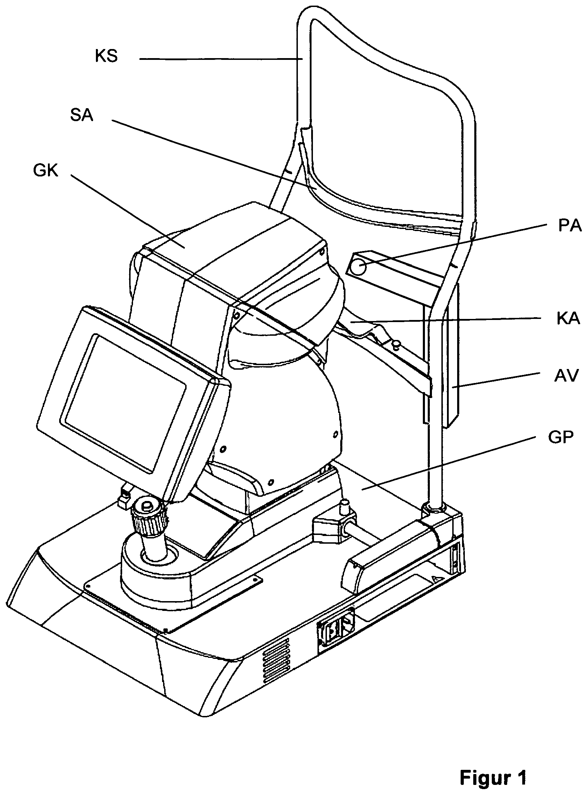

In einer ersten Ausführung zeigt **Figur 1** beispielhaft ein ophthalmologisches Messsystem in Form eines IOLMaster®, bei dem die Vorrichtung zur Aufnahme mindestens eines Kalibrier- und Prüfmittels an der Kopfstütze angeordnet ist. Der IOLMaster® besteht mindestens aus einem über eine Grundplatte **GP** mit einer Kopfstütze **KS** mit Kinnauflage **KA** zur Fixierung des zu untersuchenden Auges verbundenen Gerätemesskopf **GK,** der zur Ausrichtung auf das zu untersuchende Auge in x, y und z verstellbar ist, sowie einer nicht dargestellten Steuer- und Auswerteeinheit. Dabei sind die erforderlichen Kalibrier- und Prüfmittel in Form eines Prüfauges **PA** integriert, wobei die Kopfstütze **KS** über eine Vorrichtung **AV** zur Aufnahme mindestens eines Prüfauges **PA** verfügt. Die Vorrichtung **AV** zur Aufnahme mindestens eines Prüfauges **PA** verfügt über Mittel, mit denen das Kalibrier- und Prüfmittel mechanisch bzw. halb- oder vollautomatisch in die für die Messung erforderliche Position bewegt wird. Die Bewegung kann dabei, wie in **Figur 1** gezeigt durch Schwänken oder Kippen, aber auch durch Drehen oder lineares Ausfahren erfolgen. Vorzugsweise verfügt die Vorrichtung **AV** dafür über entsprechende Stellantriebe zur vollautomatischen Bewegung.

Weiterhin ist es vorteilhaft, dass die Vorrichtung **AV** so ausgebildet ist, dass das Prüfauge **PA** austauschbar ist. Zur eindeutigen Identifizierung des Prüfauges **PA** und einer eindeutigen Zuordenbarkeit der Messdaten verfügt dieses über eine Seriennummer, die elektrisch, optisch und/oder opto-elektrisch erfassbar ist. In einer weiteren vorteilhaften Ausgestaltung kann die Identifizierung des Prüfauges **PA** über dessen Seriennummer mittels eines RFID-Systems (radio frequency identification) erfolgen. Erfindungsgemäß verfügt das Kalibrier- und Prüfmittel über eine maschinenlesbare Kennung, die die individuellen physikalischen Daten des Kalibrier- und Prüfmittel enthält. Zusätzlich kann die maschinenlesbare Kennung die für die Kalibrierung und/oder Justierung des ophthalmologischen Messsystems erforderlichen Toleranzen und/oder Informationen welchem ophthalmologischen Messsystem das Kalibrier- und Prüfmittel zuzuordnen ist, enthalten. Hierbei ist die maschinenlesbare Kennung des Kalibrier- und Prüfmittels vorzugsweise ein Barcode, ein Data Matrix Code, ein RFID-Chip oder ein ähnlicher elektronischer Speicher. Dabei ist es unerheblich, ob das ophthalmologische Messsystem auf einem interferometrischen Messverfahren oder der Wellenfrontanalyse basiert.

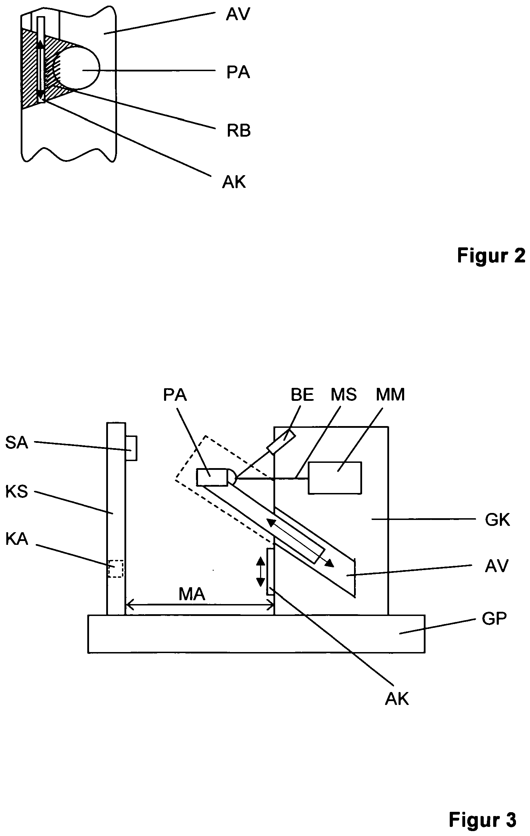

Um sicher zu stellen, dass sowohl die Kalibrierung als auch die Überprüfung der Funktion und des Kalibrierungszustandes den entsprechenden Normen entsprechen, muss gewährleistet werden, dass das verwendete Prüfauge **PA** weder verschmutzt noch beschädigt ist. Dazu zeigt die **Figur 2** eine weitere vorteilhafte Ausgestaltung, bei der die Vorrichtung **AV** zur Aufnahme mindestens eines Prüfauges **PA** über eine Abdeckklappe **AK** zum Schutz vor Beschädigungen und über Mittel zum Reinigen **RB** verfügt. In einfachsten Fall kann das Mittel zum Reinigen **RB** des Prüfauges **PA** eine Bürste sein, die an der Abdeckklappe **AK** angeordnet ist und beim Öffnen der Klappe über das Prüfauge **PA** fährt und dieses reinigt. Neben mechanischen sind aber auch pneumatische und/oder akustische Mittel zum Reinigen des Prüfauges **PA** verwendbar. Weiterhin bevorzugt ist die Verwendung schmutzabweisender Beschichtungen oder Oberflächenstrukturen auf berührbaren, optischen Oberflächen des Messsystems, insbesondere des Kalibrier- und Prüfmittels. Derartige Beschichtungen sind beispielsweise in JP 62080603 A beschrieben.

Zur Vermeidung von Verletzungen ist für die Zeit der Kalibrierung sowie der Überprüfung der Funktionsfähigkeit und des Kalibrierungszustandes ist zwingend zu gewährleisten, dass kein Patient seinen Kopf auf der Kopfstütze abgelegt hat. Dazu verfügt das ophthalmologische Messsystem zusätzlich über Sicherheitseinrichtungen, die ein Bewegen des Prüfauges **PA** verhindern, wenn ein Patient seinen Kopf bereits auf der Kopfstütze abgelegt hat. Als Sicherheitseinrichtungen können beispielsweise Lichtschranken oder auch Drucksensoren oder kapazitive Sensoren an Kinn- und/oder Stirnstütze verwendet werden.

In einer weiteren sehr vorteilhaften Ausgestaltung kann die oben beschriebene Vorrichtung **AV** zur Aufnahme mindestens eines Prüfauges **PA** mit all seinen beschriebenen vorteilhaften Ausgestaltungen in eine motorisierte Patientenaufnahme für Geräte zur Augenuntersuchung oder -behandlung, gemäß US 7,401,921 B2 integriert werden. Dies hat den Vorteil, dass die für die Bewegung der Patientenaufnahme vorgesehenen Antriebe auch für eine Positionierung des Prüfauges **PA** verwendet werden können.

Durch die einfache und kompakte Bauart, der in der US 7,401,921 B2 beschriebenen motorisierten Patientenaufnahme, lässt sich diese mit einer Vielzahl ophthalmologischer Geräte, wie beispielsweise Refraktometern, Keratometern, Funduskameras, Hornhaut-Topographen, OCT- basierten Bildaufnahmesystemen, Wellenfront-Sensoren, Laser-Augenchirurgie-Systeme und so weiter kombinieren.

In einer zweiten Ausführung zeigt **Figur 3** beispielhaft ein ophthalmologisches Messsystem bei dem die Vorrichtung zur Aufnahme mindestens eines Kalibrier- und Prüfmittels am Gerätemesskopf selbst angeordnet ist. Das ophthalmologische Messsystem besteht mindestens aus einem über eine Grundplatte **GP** mit einer Kopfstütze **KS** mit Kinnauflage **KA** zur Fixierung des zu untersuchenden Auges verbundenen Gerätemesskopf **GK,** der zur Ausrichtung auf das zu untersuchende Auge in x, y und z verstellbar ist, sowie einer nicht dargestellten Steuer- und Auswerteeinheit.

Die erforderlichen Kalibrier- und Prüfmittel in Form eines Prüfauges **PA** sind hierbei integriert, wobei der Gerätemesskopf **GK** über eine Vorrichtung **AV** zur Aufnahme mindestens eines Prüfauges **PA** verfügt. Auch hier verfügt die Vorrichtung **AV** zur Aufnahme mindestens eines Prüfauges **PA** über Mittel, mit denen das Kalibrier- und Prüfmittel mechanisch bzw. halb- oder vollautomatisch in die für die Messung erforderliche Position bewegt wird. Die Bewegung erfolgt hierbei vorzugsweise durch lineares Ausfahren, kann aber auch durch Schwänken, Kippen oder Drehen realisiert werden. Vorzugsweise verfügt die Vorrichtung **AV** dafür über entsprechende Stellantriebe zur vollautomatischen Bewegung.

Zusätzlich zeigt die **Figur 3** das Prüfauge **PA** im ausgefahrenen Zustand, d. h. in Messstellung für eine Kalibrierung bzw. Überprüfung der Funktionsfähigkeit oder des Kalibrierungszustandes.

Nach Ausfahren und/oder Ausrichten des Prüfauges **PA** wird dieses von einer Beleuchtungseinheit **BE** beleuchtet. Der von der Teststruktur des Prüfauges **PA** reflektierte Messstrahl **MS** wird von einem Messmodul **MM** erfasst und ausgewertet. Anhand des Vergleiches der ermittelten Daten mit den hinterlegten Kalibrierungsdaten wird festgestellt, ob eventuell vorhandene Abweichungen innerhalb der festgelegten Toleranzen liegen.

Um Verschmutzungen oder Beschädigungen des Prüfauges **PA** zu verhindern verfügt die Vorrichtung **AV** zur Aufnahme mindestens eines Prüfauges **PA** über eine Abdeckklappe **AK** zum Schutz vor Beschädigungen und über (nicht dargestellte) Mittel zum Reinigen verfügt. Die Mittel zum Reinigen können hierbei mechanisch, pneumatisch und/oder akustisch arbeiten.

Zur Vermeidung von Verletzungen ist für die Zeit der Kalibrierung sowie der Überprüfung der Funktionsfähigkeit und des Kalibrierungszustandes auch hier zwingend zu gewährleisten, dass kein Patient seinen Kopf auf der Kopfstütze abgelegt hat. Dazu verfügt das ophthalmologische Messsystem zusätzlich über Sicherheitseinrichtungen, die ein Bewegen des Prüfauges **PA** verhindern, wenn ein Patient seinen Kopf bereits auf der Kopfstütze abgelegt hat. Als Sicherheitseinrichtungen können beispielsweise Lichtschranken oder auch Drucksensoren oder kapazitive Sensoren an der Kopfstütze **KS** oder der Kinnauflage **KA** verwendet werden.

Die Integration der Vorrichtung **AV** zur Aufnahme mindestens eines Prüfauges **PA** in den Gerätemesskopf **GK** wirkt sich dahingehend vorteilhaft aus, dass der Abstand des Prüfauges **PA** unabhängig von der Stellung der Kopfstütze **KS** ist. Dadurch ist es möglich vor dem Ausfahren des Prüfauges **PA** den Abstand zwischen Gerätemesskopf **GK** und Kopfstütze **KS** auf einen Mindestabstand **MA** zu vergrößern, dass die Verletzung eines Patienten bereits dadurch ausgeschlossen werden kann. Auf Sicherheitseinrichtungen könnte damit auch verzichtet werden.

In einer besonderen Ausgestaltung ist die Vorrichtung zum Aufnehmen und Verändern der Position des Kalibrier- und Prüfmittels in Bezug auf die Mittel zur Erfassung zurück gestreuter oder reflektierter Lichtanteile so ausgebildet, dass neben der Vorrichtung zum Aufnehmen des Kalibrier- und Prüfmittels, Mittel zur Bewegung eines zusätzlichen optischen Elementes vorhanden sind, um das Kalibrier- und Prüfmittel in Bezug auf die Mittel zur Erfassung der zurück gestreuter oder reflektierter Lichtanteile auszurichten.

Als zusätzliches optisches Element wird hierbei im einfachsten Fall ein Planspiegel verwendet, der in den Messstrahlengang bewegt wird und diesen in Richtung des Kalibrier- und Prüfmittels umlenkt. Die Bewegung erfolgt auch hier mechanisch bzw. halb- oder vollautomatisch. Das Kalibrier- und Prüfmittel ist hierbei in einem solchen Abstand angeordnet, in dem sich üblicherweise das zu untersuchende Patientenauge befindet.

Eine derartige Anordnung hat den Vorteil, dass das Kalibrier- und Prüfmittel in das ophthalmologische Messsystem integriert werden kann und nicht bewegt werden muss. Durch einen um 45° geneigt angeordneten Planspiegel wird der Messstrahlengang um 90° abgewinkelt, so dass das Kalibrier- und Prüfmittel beispielsweise in die Gerätebasis integriert werden kann.

Das Kalibrier- und Prüfmittel verfügt auch bei dieser Ausgestaltungsvariante mindestens eine gekrümmte Oberfläche als Teststruktur und über eine Seriennummer, die elektrisch, optisch und/oder opto-elektrisch erfassbar ist, so dass die Messdaten eindeutig zuordenbar sind.

Die Vorrichtung zur Aufnahme mindestens eines Kalibrier- und Prüfmittels ist so ausgebildet, dass das Kalibrier- und Prüfmittel austauschbar ist und verfügt vorzugsweise über eine Abdeckklappe zum Schutz des Kalibrier- und Prüfmittels und/oder über Mittel zum Reinigen des Kalibrier- und Prüfmittels.

Mit der erfindungsgemäßen Anordnung wird ein ophthalmologisches Messsystem, insbesondere zur Bestimmung der biometrischen Daten eines Auges, zur Verfügung gestellt, welches die im Stand der Technik erwähnten Nachteile nicht aufweist. Die Lösung ist insbesondere für die Anwendung in ophthalmologischen Geräten mit Strukturprojektion, insbesondere Keratometern geeignet.

Die vorgeschlagene Lösung ermöglicht eine automatisierte Überprüfung von Kalibrierzustand und Funktionalität ophthalmologischer Messsysteme, die bisher ein manuelles Arbeiten mit einem Testauge erforderten. Dabei kann die Vermessung von Testaugen standardmäßig als Teil der Geräteinitialisierung konzipiert werden.

Außerdem werden Lösungen realisiert, die keine Pflegemaßnahmen (Putzen) am Testauge erfordern und ein Vertauschen oder Fehler beim Ablesen von Kalibrierdaten unmöglich macht.

Das verwendete Kalibrier- und Prüfmittel zur Kalibrierung und/oder Justierung eines ophthalmologischen Messsystems verfügt über eine maschinenlesbare Kennung, die die individuellen physikalischen Daten des Kalibrier- und Prüfmittels enthält.

Unter den individuellen physikalischen Daten, die den Erwartungswerten einer Messung mit dem Kalibrier- und Prüfmittel entsprechen, sind hierbei sowohl die physikalischen Eigenschaften hinsichtlich Form, Abmessungen, Krümmungen, Material u. ä. als auch die optischen Eigenschaften, wie beispielsweise in Bezug auf Brechung, Reflexion, Transmission und Polarisation, usw. zu verstehen.

Zusätzlich kann die maschinenlesbare Kennung auch Informationen zu den zu realisierenden Positionieraufgaben enthalten um die Kalibrierung und Prüfung weiter zu vereinfachen und zu automatisieren.

Damit kann realisiert werden, dass die Vorrichtung zur Aufnahme mindestens eines Kalibrier- und Prüfmittels und zu dessen Positionierung für verschiedene ophthalmologische Messsysteme und deren unterschiedliche Messanforderungen Verwendung finden kann. Dies hat Vorteile hinsichtlich einer kostengünstigen Herstellung, niedriger Entwicklungskosten, sowie einfacherer Montage und Reparatur.

In einer ersten vorteilhaften Ausgestaltung enthält die maschinenlesbare Kennung des Kalibrier- und Prüfmittels zusätzlich die für die Kalibrierung und/oder Justierung des ophthalmologischen Messsystems erforderlichen Toleranzen und/oder Informationen welchem ophthalmologischen Messsystem das Kalibrier- und Prüfmittel zuzuordnen ist.

In einer zweiten vorteilhaften Ausgestaltung ist die maschinenlesbare Kennung in Form eines Barcodes, einer Data Matrix Code o. ä. am Kalibrier- und Prüfmittel angeordnet.

Als Barcode, der auch als Strich- oder Balkencode bezeichnet wird, ist eine optoelektronisch lesbare Schrift zu verstehen, die aus verschieden breiten, parallelen Strichen und Lücken besteht. Die Daten aus diesem Barcode werden mit optischen Lesegeräten, wie z. B. Barcodelesegeräten (Scannern) oder Kameras, maschinell eingelesen und elektronisch weiterverarbeitet. Bei einem Barcode handelt es sich um einen eindimensionalen Code (1D-Code).

Im Gegensatz dazu ist der Data Matrix Code ein zweidimensionaler Code (2D-Code), wodurch die Informationsdichte pro Flächeneinheit deutlich erhöht werden kann. Für den Data Matrix Code existieren verschiedene Code-Schemata, wobei das Codeschema "ECC 200" aufgrund seiner sicheren Lesbarkeit am weitesten verbreitet ist. Bei diesem Codeschema werden die Daten auf einer definierten, quadratischen oder rechteckigen Fläche als Muster von Quadraten kodiert.

In einer besonders vorteilhaften Ausgestaltung ist das Kalibrier- und Prüfmittel ein Prüfauge mit Barcode, welches zur Kalibrierung und/oder Justierung eines auf einem interferometrischen Messverfahren oder der Wellenfrontanalyse basierenden, ophthalmologischen Messsystems Verwendung findet.

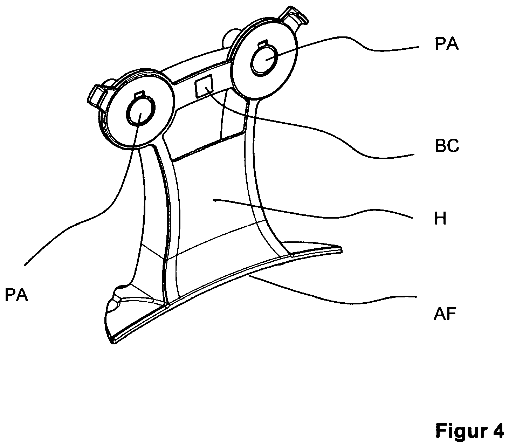

Hierzu zeigt die **Figur 4** das verwendete Kalibrier- und Prüfmittel in Form zweier, in einem Halter **H** angeordneten Prüfaugen **PA.** Der Halter **H** verfügt dabei über eine Auflagefläche **AF,** zur definierten Anordnung des Halters **H** an dem zu kalibrierenden und/oder zu justierenden, ophthalmologischen Messsystem. Die maschinenlesbare Kennung in Form eines Barcodes **BC** ist hierbei am Halter **H** angeordnet. Hierbei wird der Halter **H** mit den angeordneten Prüfaugen **PA** mechanisch per Hand auf der Kinnauflage **KA** fixiert und ein Prüfauge **PA** so in eine für die Messung erforderliche Position gebracht.

Das verwendete Kalibrier- und Prüfmittel lässt sich vorzugsweise zur Kalibrierung und/oder Justierung eines sogenannten IOLMaster® verwenden. Der IOLMaster® von der Carl Zeiss Meditec AG ist ein auf kurzkohärenten Verfahren basierendes optisches Messgerät, mit dem sich die Achslänge, Vorderkammertiefe und Hornhautbrechkraft eines Auges exakt und berührungsfrei bestimmen lässt. Zur Kalibrierung und/oder Justierung IOLMaster® wird hierbei ein Kalibrier- und Prüfmittel in Form eines Prüfauges **PA** mit Barcode, Data Matrix Code, RFID-Chip oder eines ähnlichen elektronischen Speichers verwendet.

Die maschinenlesbare Kennung in Form von Seriennummern, Barcodes, Data Matrix Codes, eines RFID-Chips oder eines ähnlichen elektronischen Speichers kann hierbei automatisiert mechanisch, elektrisch, magnetisch, optisch und/oder elektromagnetisch erfassbar sein, so dass die Messdaten eindeutig zuordenbar sind, insbesondere um die Notwendigkeit einer fehleranfällige Interaktion mit dem Nutzer des Messsystems zu vermeiden. Beispielsweise kann die Seriennummer binär kodiert in Form von Leiterbrücken zwischen elektrischen Kontakten am Sockel. Alternativ oder optional kann das das Kalibrier- und Prüfmittel auch die Kalibrierdaten selbst in einem beispielsweise elektronisch auslesbaren Speicher beinhalten.

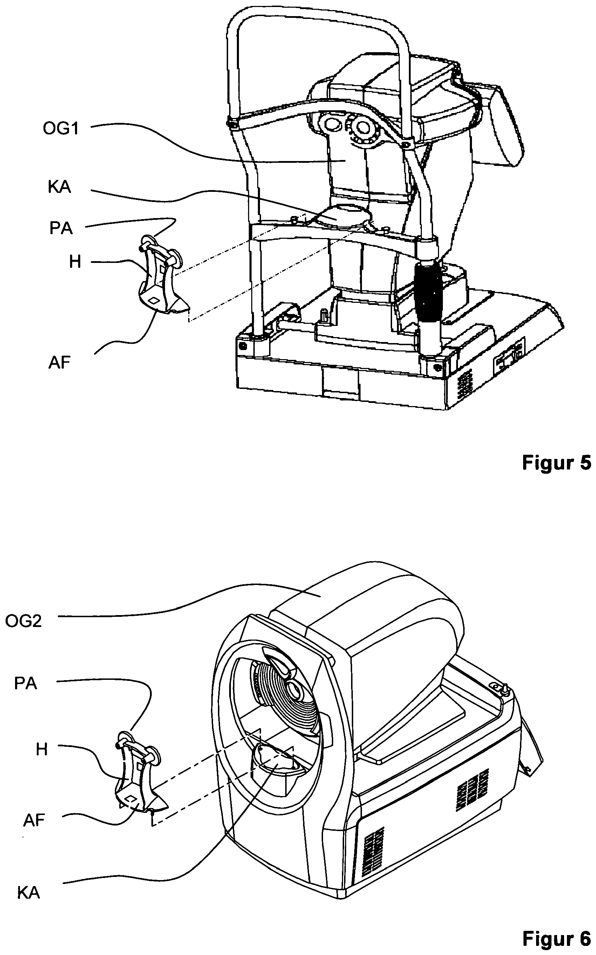

Hierzu zeigt **Figur 5** Prüfaugen **PA** nach **Figur 4** in Verbindung mit einem auf interferometrischen Messverfahren basieren, ophthalmologischen Gerät **IOL.** Wie über die gestrichelte Linie angedeutet, wird der Halter **H** mit seiner Auflagefläche **AF** auf der Kinnauflage **KA** des auf interferometrischen Messverfahren basieren, ophthalmologischen Gerätes **OG1** angeordnet.

Wie für den IOLMaster® ist das erfindungsgemäße Kalibrier- und Prüfmittel beispielsweise auch zur Kalibrierung und/oder Justierung des sogenannten i.Profiler® der Carl Zeiss Meditec AG geeignet. Im Gegensatz zum IOLMaster® basiert der i.Profiler® auf der sogenannten Wellenfront-Technologie und erstellt höchst individuelle Sehprofile der Augen. Der i.Profiler® ist nicht nur ein Keratometer und Autorefraktometer, sondern auch zusätzliches Aberrometer und ein Hornhaut-Topographiesystem.

Hierzu zeigt **Figur 6** Prüfaugen **PA** nach **Figur 4** in Verbindung mit einem auf der Wellenfrontanalyse basieren, ophthalmologischen Gerät OG2. Auch hier wird, wie über die gestrichelte Linie angedeutet, der Halter H mit seiner Auflagefläche **AF** auf der Kinnauflage **KA** des auf der Wellenfrontanalyse basieren, ophthalmologischen Gerätes **OG2** angeordnet.

Bei der Verwendung des Kalibrier- und Prüfmittels zur Kalibrierung und/oder Justierung ophthalmologischer Messsysteme wirkt sich besonders vorteilhaft aus, dass in dem ophthalmologischen Messsystem keinerlei Daten zu verwendender Kalibrier- und Prüfmittel gespeichert sein müssen. Sowohl die Kalibrierung als auch die Justierung der ophthalmologischen Messsysteme erfolgt ausschließlich anhand der in der maschinenlesbaren Kennung enthaltenen, individuellen physikalischen Daten des Kalibrier- und Prüfmittels. Voraussetzung ist lediglich, dass die verwendeten Kalibrier- und Prüfmittel für das zu kalibrierende und/oder zu justierende, ophthalmologische Messsystem geeignet sind.

Bei dem erfindungsgemäßen Verfahren gemäß des Anspruchs 12 zur Kalibrierung und/oder Justierung ophthalmologischer Messsysteme, unter Zuhilfenahme mindestens eines Kalibrier- und Prüfmittels wird das zu verwendende Kalibrier- und Prüfmittel in die Messposition gebracht, dessen individuellen physikalischen Daten von dem zu kalibrierenden und/oder zu justierenden, ophthalmologischen Messsystems ausgelesen, dessen physikalischen Eigenschaften von dem zu kalibrierenden und/oder zu justierenden, ophthalmologischen Messsystems gemessen, die ausgelesenen physikalischen Daten des Kalibrier- und Prüfmittels mit dessen gemessenen physikalischen Eigenschaften verglichen und daraus Erkenntnisse zum Kalibrier- und/oder Justierzustand sowie eine Entscheidung zur weiteren Verwendung des ophthalmologischen Messsystems abgeleitet. Dazu wird ein Kalibrier- und Prüfmittel verwendet, welches über eine maschinenlesbare Kennung verfügt, die dessen individuelle physikalische Daten enthält.

Zur Kalibrierung wird vom Bedienpersonal ein Kalibrier- und Prüfmittel mit Barcode, Data Matrix Code, RFID-Chip oder eines ähnlichen elektronischen Speichers in die entsprechende Halterung des IOLMaster® gelegt und der Messvorgang ausgelöst. Nach dem Auslesen der maschinenlesbaren Kennung des Kalibrier- und Prüfmittels werden die Messdaten am Kalibrier- und Prüfmittel bestimmt und das Ergebnis in Bezug auf die maschinenlesbare Kennung des Kalibrier- und Prüfmittel als auch der gefundenen Messabweichungen dem Bedienpersonal zur abschließenden Entscheidung dargestellt. In einem weiteren Schritt können diese Ergebnisse dokumentiert, weiterverarbeitet, zur Justierung genutzt oder aber einem automatischen Verwendungsentscheid zugeführt werden.

In einer ersten bevorzugten Ausgestaltung des Verfahrens erfolgt das Auslesen der individuellen physikalischen Daten des Kalibrier- und Prüfmittels und das Messen seiner physikalischen Eigenschaften hierbei mit nur einer optischen Messvorrichtung und zwar dem ohnehin im zu kalibrierenden und/oder zu justierenden, ophthalmologischen Messsystems vorhandenen optischen Bildverarbeitungssystem.

Allerdings ist es prinzipiell auch möglich für das Auslesen der individuellen physikalischen Daten aus der maschinenlesbaren Kennung des Kalibrier- und Prüfmittels eine separate, auf der optischen Bildverarbeitung basierende, optische Messvorrichtung zu verwenden. Das Messen der physikalischen Eigenschaften des Kalibrier- und Prüfmittels erfolgt mit ohnehin im ophthalmologischen Messsystem vorhandenen optischen Messvorrichtungen, die ebenfalls auf der optischen Bildverarbeitung basiert.

In einer zweiten bevorzugten Ausgestaltung des Verfahrens können sowohl die ermittelten physikalischen Daten als auch die Messwerte dem Nutzer zur Verfügung gestellt werden, wobei dies vorzugsweise in identischen, physikalischen Größen erfolgt. Zusätzlich können dem Nutzer zu den ermittelten physikalischen Daten und Messwerten des Kalibrier- und Prüfmittels auch Vorschläge für Toleranzlagen und/oder Verwendungsentscheide unterbreitet werden.

Allerdings ist es hierbei auch möglich, dass der Verwendungsentscheid über eine korrekte Kalibrierung vollständig automatisch verarbeitet wird und je nach Einstellung des ophthalmologischen Messsystems dieser Verwendungsentscheid zu einer Sperrung des ophthalmologischen Messsystems oder nur bestimmter Funktionen sowie weiterführender Maßnahmen führt.

Andererseits ist es selbstverständlich auch möglich, dass die Justage des ophthalmologischen Messsystems anhand der ermittelten physikalischen Daten und der Messwerten des Kalibrier- und Prüfmittels automatisch durchgeführt wird.

In einer dritten bevorzugten Ausgestaltung des Verfahrens wird das zu kalibrierende und/oder zu justierende, ophthalmologische Messsystem in den Kalibrier- bzw. Justiermodus versetzt, nachdem das zu verwendende Kalibrier- und Prüfmittel in die Messposition gebracht wurde. Die Zyklen für die von Zeit zu Zeit erforderliche Kalibrierung und/oder Justierung kann hierbei vom ophthalmologischen Messsystem anhand der verstrichenen Zeit oder auch der Anzahl der durchgeführten Messungen bestimmt und dem Nutzer angezeigt werden.

Hierbei kann das Umschalten des ophthalmologischen Messsystems in den Kalibrier- bzw. Justiermodul bei Erkennen der maschinenlesbaren Kennung des zu verwendenden Kalibrier- und Prüfmittel durch die optische Messvorrichtung automatisch erfolgen. Dazu braucht das Kalibrier- und Prüfmittel nur in die Messposition gebracht zu werden.

Mit der erfindungsgemäßen Lösung wird ein Kalibrier- und Prüfmittel zur Kalibrierung und/oder Justierung ophthalmologischer Messsysteme zur Verfügung gestellt, mit der die Kalibrierung und/oder Justierung für das Bedienpersonal wesentlich vereinfacht wird.

Außerdem werden die nach dem Stand der Technik bekannten Fehlerquellen, insbesondere eine Verwechslung oder Fehlentscheidung infolge mangelnder Sorgfalt ausgeschlossen.

Eine Verwendung falscher Kalibrier- und Prüfmittel bzw. eine falsche Zuordnung zu den hinterlegten Referenzdaten ist damit völlig ausgeschlossen. Bei jeder Kalibrierung und/oder Justierung werden die individuellen Daten des Kalibrier- und Prüfmittels aus dessen maschinenlesbarer Kennung ausgelesen, so dass zu Fehldiagnosen und sogar Fehlbehandlungen führende, und durch Kalibrier-Fehlentscheidungen bedingte, falsche Messergebnisse unwahrscheinlich sind.

Mit dem vorgeschlagenen Verfahren sind Kalibrierung und/oder Justierung auf sehr komfortable Weise möglich, so dass das Bedienpersonal auch diese Arbeit nicht zu scheuen braucht, was letztlich auch der Erhöhung der Sicherheit des Gerätes dient.

Mit der erfindungsgemäßen Lösung wird weiterhin ein erheblicher Beitrag zur Wirksamkeit vorgeschriebener Kalibrierung, unabhängig vom Ausbildungsstand des Bedienpersonals, als auch zur störungsfreien Nutzung des technischen Gerätes geleistet.

## Patentansprüche

1. Ophthalmologisches Messsystem, insbesondere zur Gewinnung biometrischen Daten eines Auges, mindestens bestehend aus Mitteln zur Beleuchtung eines Auges mit Licht und Mitteln zur Erfassung und Auswertung zurück gestreuter oder reflektierter Lichtanteile, sowie einer Steuereinheit, wobei dessen Gerätemesskopf (GK) über eine Grundplatte (GP) mit einer Kopfstütze (KS) mit Kinnauflage (KA) verbundenen ist, **dadurch gekennzeichnet, dass** für die Überprüfung der Funktion und des Kalibrierungszustandes des Messsystems mindestens ein in das ophthalmologische Messsystem integriertes Kalibrier- und Prüfmittel vorgesehen ist, welches mindestens eine Teststruktur aufweist und dass eine Vorrichtung vorgesehen ist, um das Kalibrier- und Prüfmittel aufzunehmen und seine Position in Bezug auf die Mittel zur Erfassung der zurück gestreuten oder reflektierten Lichtanteile zu verändern, wobei die Vorrichtung zur Aufnahme mindestens eines Kalibrier- und Prüfmittels an der Kopfstütze (KS) oder am Gerätemesskopf (GK) selbst angeordnet ist,
**dadurch gekennzeichnet, dass**
das mindestens eine Kalibrier- und Prüfmittel über eine maschinenlesbare Kennung verfügt, die die individuellen physikalischen Daten des Kalibrier- und Prüfmittels enthält, und dass
in dem ophthalmologischen Messsystem eine entsprechende Vorrichtung zum Aktivieren und/oder Auslesen der maschinenlesbaren Kennung vorhanden ist.

2. Ophthalmologisches Messsystem nach Anspruch 1, **dadurch gekennzeichnet, dass** die Vorrichtung zur Aufnahme mindestens eines Kalibrier- und Prüfmittels über eine Schnittstelle zur Identifizierung unterschiedlicher Kalibrier- und Prüfmittel verfügt.

3. Ophthalmologisches Messsystem nach den Ansprüchen 1 und 2, **dadurch gekennzeichnet, dass** zur mechanischen bzw. halb- oder vollautomatischen Ausrichtung des Kalibrier- und Prüfmittels auf das ophthalmologische Messsystem und zur Positionierung einer Kopfstütze (KS) und/oder Kinnauflage (KA) nur eine Antriebseinheit vorhanden ist.

4. Ophthalmologisches Messsystem nach Anspruch 3, **dadurch gekennzeichnet, dass** diese Antriebseinheit für die Positionierung der Kopfstütze (KS) und/oder Kinnauflage (KA) eine lineare und für die Ausrichtung des Kalibrier- und Prüfmittels eine lineare und/oder rotierende Bewegung realisiert.

5. Ophthalmologisches Messsystem nach Anspruch 1, **dadurch gekennzeichnet, dass** eine Vorrichtung zur Aufnahme (AV) mindestens eines Kalibrier- und Prüfmittels vorhanden ist und über eine Abdeckklappe (AK) zum Schutz und/oder über Mittel zum Reinigen (RB) des Kalibrier- und Prüfmittels verfügt.

6. Ophthalmologisches Messsystem nach Anspruch 1, **dadurch gekennzeichnet, dass** das ophthalmologische Messsystem zusätzlich über Sicherheitseinrichtungen in Form von Lichtschranken oder auch Drucksensoren oder kapazitive Sensoren an Kinn- und/oder Stirnstütze verfügt, die ein Bewegen des integrierten Kalibrier- und Prüfmittels verhindern, wenn sich der Kopf eines Patienten auf der Kopfstütze (KS) befindet.

7. Ophthalmologisches Messsystem nach Anspruch 1, **dadurch gekennzeichnet, dass** das die maschinenlesbare Kennung des Kalibrier- und Prüfmittels zusätzlich die für die Kalibrierung und/oder Justierung des ophthalmologischen Messsystems erforderlichen Toleranzen und/oder Informationen welchem ophthalmologischen Messsystem das Kalibrier- und Prüfmittel zuzuordnen ist, enthält

8. Ophthalmologisches Messsystem nach Anspruch 1, **dadurch gekennzeichnet, dass** die maschinenlesbare Kennung des Kalibrier- und Prüfmittels ein Barcode, ein Data Matrix Code, ein RFID-Chip, ein elektronischer Speicher o. ä. ist.

9. Ophthalmologisches Messsystem nach Anspruch 1, **dadurch gekennzeichnet, dass** die Vorrichtung zum Aktivieren und/ oder Auslesen der maschinenlesbaren Kennung eine optische Messvorrichtung zum Auslesen der maschinenlesbarer Kennung ist.

10. Ophthalmologisches Messsystem nach Anspruch 9, **dadurch gekennzeichnet, dass** die optische Messvorrichtung dem ohnehin vorhandenen optischen Bildverarbeitungssystem des ophthalmologischen Messsystems oder einer separaten, auf der optischen Bildverarbeitung basierende, optische Messvorrichtung entspricht.

11. Ophthalmologisches Messsystem nach den Ansprüchen 1 und 2, **dadurch gekennzeichnet, dass** das Kalibrier- und Prüfmittel ein Prüfauge (PA) mit Barcode (BC) ist und zur Kalibrierung und/oder Justierung ophthalmologischer Messsysteme Verwendung findet, die auf einem interferometrischen Messverfahren oder der Wellenfrontanalyse basieren.

12. Verfahren zur Kalibrierung und/oder Justierung eines ophthalmologischen Messsystems, wobei das zu verwendende Kalibrier- und Prüfmittel in die Messposition gebracht wird, dessen individuellen, in Form einer maschinenlesbaren Kennung vorhandenen, physikalischen Daten von dem zu kalibrierenden und/oder zu justierenden, ophthalmologischen Messsystems ausgelesen werden, dessen physikalischen Eigenschaften von dem zu kalibrierenden und/oder zu justierenden, ophthalmologischen Messsystems gemessen werden, die ausgelesenen physikalischen Daten des Kalibrier- und Prüfmittel mit dessen gemessenen physikalischen Eigenschaften verglichen werden und daraus Erkenntnisse zum Kalibrier- und/oder Justierzustand sowie eine Entscheidung zur weiteren Verwendung des ophthalmologischen Messsystems abgeleitet werden.

13. Verfahren nach Anspruch 12, **dadurch gekennzeichnet, dass** das Auslesen der individuellen physikalischen Daten des Kalibrier- und Prüfmittels und das Messen seiner physikalischen Eigenschaften mit mindestens einer optischen Messvorrichtung erfolgt, die auf der optischen Bildverarbeitung basiert und dem ohnehin vorhandenen optischen Bildverarbeitungssystem des ophthalmologischen Messsystems oder einer separaten, optischen Messvorrichtung entspricht.

14. Verfahren mindestens nach den Ansprüchen 12 und 13, **dadurch gekennzeichnet, dass** die ermittelten physikalischen Daten und Messwerte dem Nutzer in identischen physikalischen Größen zur Verfügung gestellt werden.

15. Verfahren nach den Ansprüchen 12 bis 14, **dadurch gekennzeichnet, dass** das ophthalmologischen Messsystem dem Anwender zusätzlich zu den ermittelten physikalischen Daten und Messwerten des Kalibrier- und Prüfmittels auch Vorschläge für Toleranzlagen und/oder Verwendungsentscheide unterbreitet.

16. Verfahren nach den Ansprüchen 12 bis 15, **dadurch gekennzeichnet, dass** der Verwendungsentscheid über eine korrekte Kalibrierung vollständig automatisch verarbeitet wird und je nach Einstellung des ophthalmologischen Messsystems dieser Verwendungsentscheid zu einer Sperrung des ophthalmologischen Messsystems oder bestimmter Funktionen sowie weiterführender Maßnahmen führt.

17. Verfahren nach den Ansprüchen 12 bis 16, **dadurch gekennzeichnet, dass** die Justage des ophthalmologischen Messsystems anhand der ermittelten physikalischen Daten und der Messwerten des Kalibrier- und Prüfmittels automatisch durchgeführt wird.

## Claims

1. Ophthalmological measuring system, in particular for acquiring biometric data of an eye, at least consisting of means for illuminating an eye with light and means for capturing and evaluating backscattered or reflected light components, and a control unit, wherein the device measuring head (GK) thereof is connected to a headrest (KS) with chin support (KA) via a base plate (GP), **characterized in that** for testing the function and the calibration state of the measuring system at least one calibration and test means is provided which is integrated in the ophthalmological measuring system and has at least one test structure, and **in that** an apparatus is provided to receive the calibration and test means and to change the position thereof with respect to the means for capturing the backscattered or reflected light components, wherein the apparatus for receiving at least one calibration and test means is arranged at the headrest (KS) or at the device measuring head (GK) itself,
**characterized in that** the at least one calibration and test means has a machine-readable identifier that contains the individual physical data of the calibration and test means and **in that** a corresponding apparatus for activating and/or reading the machine-readable identifier is included in the ophthalmological measuring system.

2. Ophthalmological measuring system according to Claim 1, **characterized in that** the apparatus for receiving at least one calibration and test means has an interface for identifying different calibration and test means.

3. Ophthalmological measuring system according to Claims 1 and 2, **characterized in that** only one drive unit is present for the mechanical or semi-automatic or fully automatic alignment of the calibration and test means with the ophthalmological measuring system and for positioning a headrest (KS) and/or chin support (KA) .

4. Ophthalmological measuring system according to Claim 3, **characterized in that** this drive unit for the positioning of the headrest (KS) and/or chin support (KA) realizes a linear movement and for the alignment of the calibration and test means realizes a linear and/or rotating movement.

5. Ophthalmological measuring system according to Claim 1, **characterized in that** an apparatus for receiving (AV) at least one calibration and test means is present and has a covering flap (AK) for protecting and/or means for cleaning (RB) the calibration and test means.

6. Ophthalmological measuring system according to Claim 1, **characterized in that** the ophthalmological measuring system additionally has safety devices in the form of light barriers or pressure sensors or capacitive sensors at the chin rest and/or forehead rest, which prevent movement of the integrated calibration and test means if the head of the patient is located on the headrest (KS).

7. Ophthalmological measuring system according to Claim 1, **characterized in that** the machine-readable identifier of the calibration and test means additionally contains the tolerances that are required for the calibration and/or adjusting of the ophthalmological measuring system and/or the information as to to which ophthalmological measuring system the calibration and test means is to be assigned.

8. Ophthalmological measuring system according to Claim 1, **characterized in that** the machine-readable identifier of the calibration and test means is a barcode, a data matrix code, an RFID chip, an electronic memory or the like.

9. Ophthalmological measuring system according to Claim 1, **characterized in that** the apparatus for activating and/or reading the machine-readable identifier is an optical measuring apparatus for reading the machine-readable identifier.

10. Ophthalmological measuring system according to Claim 9, **characterized in that** the optical measuring apparatus corresponds to the already existing optical image processing system of the ophthalmological measuring system or to a separate optical measuring apparatus based on optical image processing.

11. Ophthalmological measuring system according to Claims 1 and 2, **characterized in that** the calibration and test means is a test eye (PA) with a barcode (BC) and is used for calibrating and/or adjusting the ophthalmological measuring systems that are based on an interferometric measuring method or wavefront analysis.

12. Method for calibrating and/or adjusting an ophthalmological measuring system, wherein the calibration and test means to be used is brought into the measurement position, the individual physical data thereof, present in the form of a machine-readable identifier, are read by the ophthalmological measuring system to be calibrated and/or to be adjusted, the physical properties thereof are measured by the ophthalmological measuring system to be calibrated and/or to be adjusted, the read physical data of the calibration and test means are compared to the measured physical properties thereof and conclusions relating to the calibration and/or adjusting state and a decision as to the further use of the ophthalmological measuring system are derived therefrom.

13. Method according to Claim 12, **characterized in that** the reading of the individual physical data of the calibration and test means and measurement of the physical properties thereof are performed with at least one optical measuring apparatus that is based on optical image processing and corresponds to the already existing optical image processing system of the ophthalmological measuring system or to a separate, optical measuring apparatus.

14. Method at least according to Claims 12 and 13, **characterized in that** the ascertained physical data and measurement values are provided to the user in identical physical variables.

15. Method according to Claims 12 to 14, **characterized in that** the ophthalmological measuring system also provides, in addition to the ascertained physical data and measurement values of the calibration and test means, suggestions for tolerance positions and/or use decisions to the user.

16. Method according to Claims 12 to 15, **characterized in that** the use decision relating to a correct calibration is provided entirely automatically and, depending on the setting of the ophthalmological measuring system, this use decision results in locking of the ophthalmological measuring system or of specific functions and further measures.

17. Method according to Claims 12 to 16, **characterized in that** the adjustment of the ophthalmological measuring system is automatically performed on the basis of the ascertained physical data and the measurement values of the calibration and test means.

## Revendications

1. Système de mesure ophtalmologique, en particulier destiné à l'acquisition de données biométriques relatives à un oeil, constitué au moins de moyens permettant d'éclairer un oeil avec de la lumière et de moyens destinés à détecter et à évaluer des composantes de lumière diffusées ou réfléchies en retour ainsi qu'une unité de commande, dans lequel la tête de mesure d'appareil (GK) dudit système de mesure est reliée par l'intermédiaire d'une plaque de base (GP) à un support de tête (KS) muni d'un appuie-menton (KA), **caractérisé en ce que**, pour tester le fonctionnement et l'état d'étalonnage du système de mesure, il est prévu au moins un moyen d'étalonnage et de test intégré au système de mesure ophtalmologique, lequel moyen comporte au moins une structure de test et **en ce qu'**il est prévu un dispositif permettant de recevoir le moyen d'étalonnage et de test et de modifier sa position par rapport aux moyens destinés à détecter les composantes de lumière diffusées ou réfléchies en retour, dans lequel le dispositif destiné à recevoir au moins un moyen d'étalonnage et de test est disposé sur le support de tête (KS) ou sur la tête de mesure d'appareil (GK) elle-même,
**caractérisé en ce que** ledit au moins un moyen d'étalonnage et de test comporte une identification lisible par machine qui contient des données physiques individuelles du moyen d'étalonnage et de test, et **en ce qu'**il est prévu dans le système de mesure ophtalmologique un dispositif destiné à activer et/ou à lire l'identification lisible par machine.

2. Système de mesure ophtalmologique selon la revendication 1, **caractérisé en ce que** le dispositif destiné à recevoir au moins un moyen d'étalonnage et de test comporte une interface destinée à identifier différents moyens d'étalonnage et de test.

3. Système de mesure ophtalmologique selon les revendications 1 et 2, **caractérisé en ce qu'**il est prévu une seule unité d'entraînement pour orienter mécaniquement ou de manière semi-automatisée ou entièrement automatisée le moyen d'étalonnage et de test sur le système de mesure ophtalmologique et pour positionner un support de tête (KS) et/ou un appuie-menton (KA).

4. Système de mesure ophtalmologique selon la revendication 3, **caractérisé en ce que** ladite unité d'entraînement réalise un mouvement linéaire pour le positionnement du support de tête (KS) et/ou de l'appuie-menton (KA) ou un mouvement linéaire et/ou de rotation pour l'orientation du moyen d'étalonnage et de test.

5. Système de mesure ophtalmologique selon la revendication 1, **caractérisé en ce qu'**il est prévu un dispositif destiné à recevoir (AV) au moins un moyen d'étalonnage et de test et **en ce qu'**il comporte un capot de protection (AK) destiné à assurer une protection et/ou des moyens destinés à nettoyer (RB) le moyen d'étalonnage et de test.

6. Système de mesure ophtalmologique selon la revendication 1, **caractérisé en ce que** le système de mesure ophtalmologique comporte en outre des dispositifs de sécurité sous la forme de barrières lumineuses ainsi que de capteurs de pression ou de capteurs capacitifs sur l'appuie-menton et/ou le support frontal, qui empêchent un mouvement du moyen d'étalonnage et de test intégré lorsque la tête d'un patient est disposée sur le support de tête (KS).

7. Système de mesure ophtalmologique selon la revendication 1, **caractérisé en ce que** l'identification lisible par machine du moyen d'étalonnage et de test contient en outre les tolérances et/ou les informations nécessaires pour l'étalonnage et/ou l'ajustement du système de mesure ophtalmologique auquel est associé le moyen d'étalonnage et de test.

8. Système de mesure ophtalmologique selon la revendication 1, **caractérisé en ce que** l'identification lisible par machine du moyen d'étalonnage et de test intégré est un code à barres, un code de type Data Matrix, une puce RFID, une mémoire électronique, ou autre.

9. Système de mesure ophtalmologique selon la revendication 1, **caractérisé en ce que** le dispositif destiné à activer et/ou à lire l'identification lisible par machine est un dispositif de mesure optique destiné à lire l'identification lisible par machine.

10. Système de mesure ophtalmologique selon la revendication 9, **caractérisé en ce que** le dispositif de mesure optique correspond au système de traitement d'image optique déjà existant du système de mesure ophtalmologique ou à un dispositif de mesure optique séparé basé sur le traitement d'image optique.

11. Système de mesure ophtalmologique selon les revendications 1 et 2, **caractérisé en ce que** le moyen d'étalonnage et de test est un oeil de test (PA) comportant un code à barres (BC) et est utilisé pour étalonner et/ou ajuster des systèmes des mesure ophtalmologiques fondés sur un procédé de mesure interférométrique ou l'analyse de fronts d'ondes.

12. Procédé pour l'étalonnage et/ou l'ajustement d'un système de mesure ophtalmologique, dans lequel le moyen d'étalonnage et de test devant être utilisé est amené à la positon de mesure à laquelle des données physiques présentes sous la forme d'une identification lisible par machine sont lues par le système de mesure ophtalmologique à étalonner et/ou à ajuster, identification dont les propriétés physiques sont mesurées par le système de mesure ophtalmologique à étalonner et/ou à ajuster, les données physiques lues du moyen d'étalonnage et de test sont comparées à ses propriétés physiques mesurées et des conclusions en sont tirées quant à l'état d'étalonnage et/ou d'ajustement et une décision en est tirée quant à la poursuite de l'utilisation du système de mesure ophtalmologique.

13. Procédé selon la revendication 12, **caractérisé en ce que** la lecture des données physiques individuelles du moyen d'étalonnage et de test et la mesure de ses propriétés physiques sont effectuées à l'aide d'au moins un dispositif de mesure optique qui est basé sur le traitement d'image optique et qui correspond au système de traitement d'image optique déjà présent du système de mesure ophtalmologique ou d'un dispositif de mesure optique séparé.

14. Procédé selon au moins l'une des revendications 12 et 13, **caractérisé en ce que** les données physiques et les valeurs de mesure déterminées sont mises à disposition de l'utilisateur sous forme de grandeurs physiques identiques.

15. Procédé selon l'une quelconque des revendications 12 à 14, **caractérisé en ce que** le système de mesure ophtalmologique présente à l'utilisateur, en plus des données physiques et des valeurs de mesure déterminées du moyen d'étalonnage et de test des suggestions de seuils de tolérance et/ou de décisions d'utilisation.

16. Procédé selon les revendications 12 à 15, **caractérisé en ce que** la décision d'utilisation relative à un étalonnage correct est traitée de manière entièrement automatique et **en ce que**, selon le réglage du système de mesure ophtalmologique, ladite décision d'utilisation conduit à un blocage du système de mesure ophtalmologique ou de fonctions déterminées ainsi qu'à d'autres interventions.

17. Procédé selon les revendications 12 à 16, **caractérisé en ce que** l'ajustement du système de mesure ophtalmologique est effectué automatiquement sur la base des données physiques et des valeurs de mesure déterminées du moyen d'étalonnage et de test.
